# EUROPEAN PATENT SPECIFICATION

(11) **EP 3 437 531 B1**
(45) Date of publication and mention of the grant of the patent: **03.02.2021**
(21) Application number: 16897094.5
(22) Date of filing: 21.12.2016
(51) Int. Cl.: A47K 10/16, A61K 8/02, A61K 8/34, A61Q 19/00, D21H 19/10, D21H 27/00, D21H 17/06, D21H 17/00

(54) **TISSUE-PAPER**
TISSUEPAPIER
PAPIER MINCE

(30) Priority: 31.03.2016 JP 2016071824
(43) Date of publication of application: 06.02.2019
(73) Proprietor: Daio Paper Corporation, Ehime 799-0492 (JP)
(72) Inventor: YASUI, Shuta, Fujinomiya-shi Shizuoka 418-0038 (JP)
(74) Representative: Held, Stephan
(86) International application number: PCT/JP2016/088026
(87) International publication number: WO 2017/168878

(56) References cited:
- EP-A1- 2 589 324
- EP-A1- 3 279 396
- JP-A- 2011 152 426
- JP-A- 2011 156 120
- JP-A- 2013 227 707
- JP-A- 2013 511 509

## Description

### Technical Field

The present invention relates to tissue paper, particularly to tissue paper containing a moisturizer.

### Background Art

Dry type tissue paper used for blowing one's nose or the like includes tissue paper called chemicals applied type, moisturizing tissue paper, or the like, and tissue paper to which chemicals are not applied, called a generic type or the like.

The chemicals applied type tissue paper enhances "soft texture" and "smoothness" because of a hygroscopic action of glycerin contained in the chemicals. Particularly, when a user blows his/her nose repeatedly, the user can actually feel that the surroundings of the nose do not hurt.

Along with the popularization of such chemicals applied type tissue paper, there is an increasing demand not only for "soft texture" and "smoothness" but also for improvement in texture such as "fullness" from consumers.

In addition, in the chemicals applied type tissue paper, raw material cost and manufacturing cost are higher than generic type tissue paper because of using chemicals, is regarded as a high added value product at a high price. In this way, some consumers refrain from purchasing the chemicals applied type tissue paper because of a higher price than a generic product.

Meanwhile, conventional chemicals applied type tissue paper exhibits "soft texture" by the hygroscopic action, which therewith enhances "moist" feeling or "wilted" feeling, and hardly enhances feeling of fullness ("fullness"). That is, in the conventional chemicals applied type tissue paper, when it is tried to maintain or improve "soft texture", "fullness" is not sufficiently exhibited disadvantageously. On the contrary, when it is tried to exhibit "fullness" and to reduce the cost, for example, by reducing the amount of a chemical liquid used, "soft feeling" is not sufficiently exhibited disadvantageously.

Furthermore, in the conventional chemicals applied type tissue paper, the "moist" feeling, the "wilted" feeling, or less "fullness" may make a user feel a feeling of contained chemicals such as "wet feeling" or "moist feeling" like a wet type sheet impregnated with a large amount of chemicals. Because of such "wet feeling" or "moist feeling", the user feels as if the chemicals adhere to the skin, for example, in blowing his/her nose with the conventional chemicals applied type tissue paper. Therefore, a consumer who is reluctant to purchase tissue paper applied with chemicals sometimes refrains from purchasing or using the chemicals applied type tissue paper while demanding a quality not causing a hurt even when blowing his/her nose.

### Citation List

### Patent Literature

Patent Literature 1: JP 2013-511509 A
Patent Literature 2: JP 2013-511508 A
Patent Literature 3: JP 2011-522133 A

EP 3 279 396 A1 describes a 2-ply tissue paper having a basis weight per ply of less than 13 g/m², a 2-ply paper thickness of less than 135 µm, and containing more than 1.4 g/m² and less than 4.9 g/m² of chemical agent which contains glycerin and 1,3-propanediol in a total amount of 83 mass% or more, wherein the mass ratio of glycerin and 1,3-propanediol is above 1 : 0.18 and less than 1 : 5.11.

EP 2 589 324 A1 relates to two-ply tissue paper including chemicals in an amount of 2.0 to 5.5 g/m², the basis weight per each layer is 10 to 25 mg/m², and the thickness of the tissue paper is 100 to 140 µm. The chemicals may include polyalcohol such as glycerin, diglycerin, propylene glycol, 1,3-butylene glycol, polyethylene glycol, and derivative thereof.

### Summary of Invention

### Technical Problem

Therefore, a main object of the present invention is to provide moisturizing agent applied tissue paper having excellent "soft texture" and "fullness", having a low load on the skin, for example, even when being frequently used for blowing one's nose, and hardly causing a user to feel the feeling of applied chemicals, at the low production cost.

### Solution to Problem

Means for solving the above problems and actions and effects thereof are as described below.

### [Invention according to claim 1]

Two-ply tissue paper,
wherein a basis weight per ply is 11.8 g/m² or more and less than 13.2 g/m² per ply,
a paper thickness in the two-ply state is more than 122 µm and less than 138 µm,
chemicals containing mainly glycerin and 1,3-propanediol are contained,
the chemicals having a mass ratio between the glycerin and the 1,3-propanediol of more than 1 :0.03 and less than 1 :0.25, are contained in an amount of 1.0 g/m² or more and less than 2.2 g/m² and
a ratio of 1,3-propanediol in the paper is more than 0.13% by mass and less than 1.70% by mass.

### [Invention according to claim 1]

The two-ply tissue paper according to claim 1, wherein MMD is less than 7.9 and softness is less than 1.09 cN/100 mm.

### (Action and effect)

The tissue paper of the present invention is 2-ply tissue paper having a basis weight of 11.8 g/m² or more and less than 13.2 g/m² per ply, uses chemicals containing mainly glycerin and 1,3-propanediol in a range of a paper thickness of more than 122 µm and less than 138 µm for the 2-ply, and particularly sets a ratio of 1,3-propanediol in the paper to more than 0.13% by mass and less than 1.70% by mass. As a result, "soft texture" equal to or higher than conventional moisturizing agent-containing tissue paper is exhibited, a load on the skin is low even when the paper cleans the skin repeatedly, for example, even when the paper is frequently used for blowing one's nose, "fullness" is improved without decreasing, and both "soft texture" and "fullness" are achieved. Furthermore, the tissue paper is moisturizing agent-containing tissue paper not causing a user to feel a feeling of applied chemicals such as "wet feeling", "moist feeling", or "sticky feeling", and causing a user to feel light and dry feeling like generic type tissue paper.

In addition, about 4.5 to 7.5 g/m² of chemicals is required, in conventionally commercially available moisturizing tissue paper, is required to exhibit "soft texture" sufficiently. However, in the tissue paper of the present invention, when a mass ratio between glycerin and 1,3-propanediol is more than 1 : 0.03 and less than 1 : 0.25, particularly even if the chemicals are contained in a very small amount of 1.0 g/m² or more and less than 2.2 g/m², sufficient "soft texture" is achieved. Therefore, it is possible to reduce the amount of the used chemicals in the manufacturing and to reduce the manufacturing cost.

### Advantageous Effects of Invention

As described above, the present invention provides moisturizing agent-containing tissue paper having excellent "soft texture" and "fullness", having a low load on the skin even when being frequently used for blowing one's nose, and hardly causing a user to feel a feeling of applied chemicals, at the low production cost.

### Brief Description of Drawings

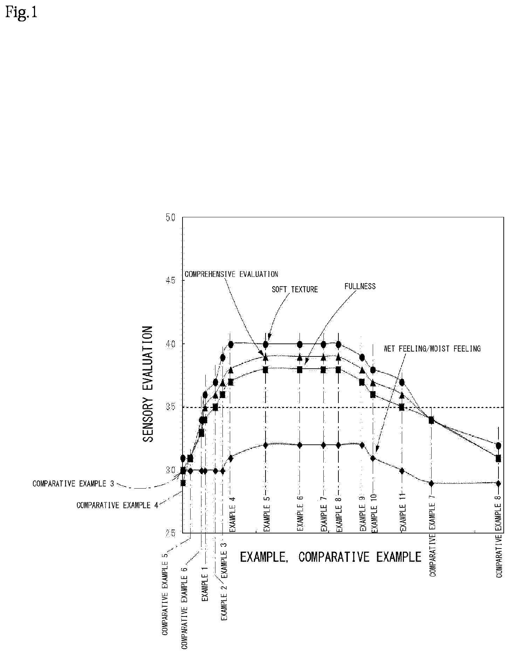

Fig. 1 is a graph illustrating results of a test example according to an embodiment.

### Description of Embodiments

Hereinafter, an embodiment of the present invention will be described.

Tissue paper according to the present embodiment is 2-ply tissue paper having a basis weight of 11.8 g/m² or more and less than 13.2 g/m² per ply, having a paper thickness of more than 122 µm and less than 138 µm for the 2-ply, and containing chemicals containing mainly glycerin and 1,3-propanediol, in which a ratio of 1,3-propanediol in the paper is more than 0.13% by mass and less than 1.70% by mass. The ratio of 1,3-propanediol in the paper is a ratio of the mass of 1.3-propanediol with respect to the mass of the tissue paper at the time of absolute dryness.

The tissue paper according to the present embodiment characteristically makes both "soft texture" and "fullness" excellent by satisfying all the conditions of the number of plies, the basis weight, the paper thickness, the composition of the chemicals, and a ratio of 1,3-propanediol in the paper. Furthermore, a feeling of applied chemicals such as "wet feeling", "moist feeling", or "sticky feeling" is reduced.

Even when the tissue paper according to the present embodiment in the above ranges of the number of plies, the basis weight, and the paper thickness, uses the chemicals containing mainly glycerin and 1,3-propanediol, if a ratio of 1,3-propanediol in the paper is outside the range of more than 0.13% by mass and less than 1.70% by mass, the tissue paper hardly exhibits "fullness", and does not sufficiently reduce the feeling of applied chemicals such as "sticky feeling" or "wet feeling". In view of this, it is clearly understood that 1,3-propanediol acts so as to suppress "sticky feeling" or the like and to cause a user to feel "fullness" while improving "soft texture" by hygroscopic action of glycerin rather than decreasing the "soft texture", and the action is remarkably exhibited particularly in the above ranges of the number of plies, the basis weight, and the paper thickness.

In addition, in the tissue paper according to the present embodiment, a mass ratio between glycerin and 1,3-propanediol in the chemicals is more than 1 : 0.03 and less than 1 : 0.25. When the mass ratio between glycerin and 1,3-propanediol is 1 : 0.03 or less, "soft texture" and "fullness" are hardly exhibited, and it may be impossible to sufficiently obtain an effect of improving "sticky feeling" or the like. In addition, when the mass ratio is 1 : 0.25 or more, the effect of improving "sticky feeling" or the like decreases, and "fullness" may also decrease. It is considered this depends on a balance between an effect mainly due to the hygroscopic action of glycerin in the effect of the whole chemicals exhibiting "soft texture", and an effect of reducing "sticky feeling" and exhibition of "fullness" by 1,3-propanediol. In the tissue paper according to the present embodiment, the mass ratio between glycerin and 1,3-propanediol is particularly preferably 1 : 0.04 or more and 1 : 0.21 or less.

Meanwhile, in the tissue paper according to the present embodiment, when the chemicals have a mass ratio of more than 1 : 0.03 and less than 1 : 0.25 between glycerin and 1,3-propanediol, the content of the chemicals is 1.0 g/m² or more and less than 2.2 g/m². When the content of the chemicals is less than 1.0 g/m², "soft texture" and "fullness" are hardly exhibited particularly in the moisturizing tissue paper. When the content of the chemicals exceeds 2.2 g/m², the feeling of applied chemicals such as "sticky feeling" or "wet feeling" is not be sufficiently reduced in some cases. This depends on a balance between the density (dense or sparse) of paper determined by the number of plies, the basis weight, and the paper thickness, and the retention amount of the chemicals as an effective component for exhibiting "soft texture" and "fullness", and furthermore, depends on the mass ratio between propanediol and glycerin in blending of glycerin exhibiting "soft texture" by the hygroscopic action and propanediol suppressing "sticky feeling" or the like while improving "soft texture" by the hygroscopic action of glycerin rather than decreasing the "soft texture". Note that the content of the chemicals of 1.0 g/m² or more and less than 2.2 g/m² is lower than the content of the chemicals of conventional commercially available moisturizing tissue paper. Therefore, in the tissue paper of the present embodiment, the amount of the used chemicals can be reduced as compared with a conventional product, and the manufacturing cost can be suppressed. In the tissue paper according to the present embodiment, 1,3-propanediol acts so as to improve "soft texture" due to the hygroscopic action of glycerin rather than decreasing the "soft texture". Furthermore, the improvement in "fullness" by 1,3-propanediol easily causes a user to feel "soft texture" synergistically. Therefore, "soft texture" equal to or higher than a conventional product is exhibited in spite of the small content of the chemicals as described above.

Furthermore, when the number of plies, the basis weight, and the paper thickness are outside the above ranges in relation to the content of the chemicals and the composition of the chemicals, "soft texture" and "fullness" are not sufficiently exhibited, and reduction in "sticky feeling" and "wet feeling" is hardly exhibited. It is considered that this is because a balance between the amount of fibers and interfiber gaps, and the chemicals is lost depending on the number of plies, the basis weight, and the paper thickness, and furthermore, the number of plies, the basis weight, and the paper thickness have a large influence on the amount of fibers largely contributing to hardness and stiffness, and therefore hard feeling or the like of the fibers themselves largely acts. Particularly, in the tissue paper according to the present embodiment, the basis weight per ply is preferably 11.8 g/m² or more and 13.0 g/m² or less, and the paper thickness is preferably 125 µm or more and 135 µm or less for the 2-ply.

As described above, the tissue paper according to the present embodiment characteristically makes both "soft texture" and "fullness" excellent by satisfying all the conditions of the number of plies, the basis weight, the paper thickness, the composition of the chemicals, and the ratio of 1,3-propanediol in the paper. Furthermore, the feeling of applied chemicals such as "wet feeling", "moist feeling", or "sticky feeling" is reduced. Besides, the amount of the used chemicals is small and the manufacturing is possible at the low cost.

Here, in the tissue paper of the present embodiment, it is necessary that each ply constituting the 2-ply contains the chemicals. More preferably, it is desirable that the chemicals are homogenized as a whole of the 2-ply tissue paper. For example, when there is an excessive difference in the content of the chemicals per basis weight between plies, "wet feeling" and "moist feeling" on one side may be strong, and soft texture may be hardly felt on the other side. In order to make the chemicals homogeneous as the whole 2-ply tissue paper, it is only required to adopt a general method for manufacturing moisturizing tissue paper to apply a chemical liquid to a base paper. That is, even when the chemicals are applied to a base paper from both sides or from one side, homogenization of the chemicals proceeds between plies in contact with each other during a subsequent seasoning period and product storage. Therefore, the chemicals are homogenized as the whole two-ply tissue paper.

Here, the basis weight in the present invention is a value measured based on JIS P 8124 (1998). The paper thickness is a value obtained by sufficiently subjecting a test piece to humidity control under conditions of JIS P 8111 (1998), and then measuring the paper thickness in a 2-ply state using a dial thickness gauge (thickness measuring instrument) "PEACOCK G type" (manufactured by Ozaki MFG. Co., Ltd.) under the same conditions. Specifically, the paper thickness is measured by confirming that there is no rubbish, dust, or the like between a plunger and a measuring table, placing the plunger on the measuring table, moving a scale of the dial thickness gauge to adjust a zero point, then raising the plunger, placing a sample on a test table, lowering the plunger slowly, and reading the current gauge. At this time, the plunger is just placed. A terminal of the plunger is made of metal, and a circular plane thereof with a diameter of 10 mm strikes perpendicularly to a paper plane, and a load at the time of measuring the paper thickness is about 70 gf. Note that the paper thickness is an average value obtained by performing the measurement 10 times.

The content of the chemicals in the present invention is the amount of the chemicals contained in the tissue paper at the time of absolute dryness. Here, the term "time of absolute dryness" refers to the amount of the chemicals in a state of being dried at a temperature of 65°C and a humidity of 10% until the chemicals obtain a constant mass.

The mass ratio between glycerin and 1,3-propanediol is a mass ratio between glycerin and 1,3-propanediol in the content of the chemicals.

The chemicals according to the present invention contain glycerin and 1,3-propanediol as a main component for exhibiting an effect as a moisturizing agent. Specifically, the total content of glycerin and 1,3-propanediol is favorably more than 67.9% by mass, and preferably 69.9% by mass or more. When the total amount of glycerin and 1,3-propanediol in the chemicals is 67.9% by mass or less, the effect of the present invention is hardly exhibited.

In the tissue paper according to the present embodiment, it is desirable that the content ratio of the chemicals is 4.5% by mass or more and 9.0% by mass or less. The content ratio of the chemicals is a ratio of the mass of the chemicals with respect to the mass of the tissue paper at the time of absolute dryness. When the content ratio of the chemicals is less than 4.5% by mass, "soft texture" and "fullness" are hardly improved. When the content ratio of the chemicals exceeds 9.0% by mass, "fullness" is hardly exhibited, and an effect of reducing the feeling of applied chemicals such as "wet feeling", "moist feeling", or "sticky feeling" is hardly exhibited.

Incidentally, the tissue paper according to the present embodiment may contain a known auxiliary agent in addition to glycerin and 1,3-propanediol in the chemicals as long as the effect of the present invention is not hindered. Examples of the auxiliary agent include a moisturizing auxiliary component such as sorbitol, a hydrophilic polymer gelling agent for enhancing retention of moisture in the tissue paper, such as glucomannan, a softener such as a surfactant or a phosphate, an oil component for aiding exhibition of smoothness, such as a liquid paraffin, an emulsifier for stabilizing a moisturizing agent and improving application properties, an antiseptic agent, and an antifoaming agent. Incidentally, a component such as the moisturizing auxiliary component or the hydrophilic polymer gelling agent for enhancing retention of moisture may decrease "fullness" when being contained excessively. Therefore, it is favorable that the content thereof is 1.0% by mass or less, preferably 0.6% by mass % or less, and more preferably 0.5% by mass or less.

Meanwhile, the tissue paper according to the present embodiment desirably has MMD of less than 7.9 and softness of less than 1.09 cN/100 mm. It is particularly preferable that the MMD is 7.7 or less and the softness is 1.03 cN/100 mm or less. MMD is one of indices of "smoothness" of a surface. If MMD is within the above range, smoothness is felt sufficiently. In addition, softness is one of indices of "soft texture". If softness is within the above range, a difference in "soft texture" from a generic type tissue paper containing no moisturizing agent is easily felt, and elasticity is also felt. In the tissue paper according to the present embodiment, the above MMD and softness can be achieved by setting the number of plies, the basis weight, the paper thickness, the composition of the chemicals, and the content of the chemicals in the present invention. Further adjustment can be made according to the composition of pulp and a crepe ratio in the manufacturing.

Note that MMD is a fluctuation of mean frictional coefficient. As a numerical value thereof is smaller, smoothness is better. As a numerical value thereof is larger, smoothness is poorer. In a method for measuring MMD, while a contact surface of a friction contactor is brought into contact with a surface of a measurement sample to which a tension of 20 g/cm is applied in a predetermined direction at a contact pressure of 25 g, the measurement sample is moved by 2 cm in substantially the same direction as the direction in which the tension is applied at a speed of 0.1 cm/s, and a friction coefficient at this time is measured using a friction tester KES-SE (manufactured by Kato Tech Co., Ltd.). A value obtained by dividing the friction coefficient by a friction distance (moving distance = 2 cm) is MMD. Incidentally, the friction contactor is formed by adjoining 20 piano wires P each having a diameter of 0.5 mm, and has a contact surface formed such that the length and the width are both 10 mm. On the contact surface, a unit bulging portion having the front end formed with 20 piano wires P (radius of curvature: 0.25 mm) is formed.

The softness is a value measured according to a handle-o-meter method according to a JIS L 1096 E method. However, a test piece has a size of 100 mm × 100 mm, and measurement is performed with a clearance of 5 mm. Measurement is performed five times in each of a longitudinal direction and a lateral direction with one ply, and an average value of all the ten values is represented in unit of cN/100 mm.

The tissue paper according to the present invention can be manufactured, for example, by adjusting glycerin, 1,3-propanediol, and an appropriate auxiliary agent so as to have a viscosity according to an application method using an appropriate solvent such as water to adjust a moisturizing chemical liquid, and applying the chemical liquid to a base paper of the tissue paper by external addition using a known chemical liquid application apparatus such as a roll transfer device including a flexo printing machine and a gravure printing machine, or a spray coating device. Incidentally, in this case, the moisturizing chemical liquid may be applied to the base paper of the tissue paper on one side. However, it is desirable that the moisturizing chemical liquid is applied on both sides, because which makes easier to achieve the uniformity in smoothness of the both sides.

A fiber material constituting the tissue paper according to the present embodiment is a pulp fiber and is desirably constituted by NBKP (Nadelholz Bleached Kraft Pulp, softwood kraft pulp) and LBKP (Laubholz Bleached Kraft Pulp, hardwood kraft pulp) used for tissue paper. The tissue paper may include waste paper pulp. However, it is extremely desirable that the tissue paper is constituted only by NBKP and LBKP which are each virgin pulp. A blend ratio is NBKP : LBKP = 25 : 75 to 40 : 60 in terms of mass ratio. Within this range, it is easy to exhibit paper strength and "soft texture" necessary for blowing one's nose.

Meanwhile, in the tissue paper according to the present embodiment, it is desirable that an elongation percentage in a longitudinal direction is 10.0% or more and 12.9% or less. When the elongation is within this range, it is easy to exhibit sufficient strength and usability at the time of use, for example, at the time of blowing one's nose. The elongation is also related to a surface property of tissue paper having a fine crepe on a surface thereof. With the above elongation, smoothness of the surface is easily felt. Note that the elongation is a value measured according to a tensile test of JIS P 8113 (1998). Examples of a measuring device include a "general-purpose tensile compression tester TG-200N" manufactured by Minebea Inc. The elongation can be adjusted by a crepe ratio at the time of manufacturing base paper of the tissue paper.

Meanwhile, the tissue paper according to the present embodiment has a dry tensile strength of 90 cN/25 mm or more and 120 cN/25 mm or less, preferably 92 cN/25 mm or more and 116 cN/25 mm or less in a cross direction (CD) of the 2-ply, has a wet tensile strength of 35 cN/25 mm or more and 60 cN/25 mm or less, preferably 40 cN/25 mm or more and 53 cN/25 mm or less in CD of the 2-ply. It is desirable that the ratio of the wet tensile strength in CD of the 2-ply with respect to the dry tensile strength in CD of the 2-ply is 0.40 or more. The CD is also referred to as a lateral direction of paper and is a direction perpendicular to a flow direction (machine direction (MD)) at the time of paper manufacturing. The dry tensile strength is defined in JIS P 8113, and the wet tensile strength is defined in JIS P 8135 (1998). With each strength described above, sufficient strength enough to withstand use can be exhibited.

In the tissue paper according to the present embodiment, the ratio of the wet tensile strength in CD of the 2-ply with respect to the dry tensile strength in CD of the 2-ply is 0.40 or more. The tissue paper according to the present embodiment has a relatively small difference in strength between a dry state and a wet state as compared with a general moisturizing tissue. With such a difference in strength, for example, particularly when blowing one's nose, a user feels "toughness (strength and a feeling of relief)" in a usage mode changing from a dry state to a wet state. Furthermore, it is difficult for the user to feel a change in strength of the paper in such a usage mode, and the user does not feel discomfort that "smoothness" changes during use.

In the tissue paper according to the present embodiment, in order to adjust the dry tensile strength and the wet tensile strength to the above values, a dry paper strengthening agent or a wet paper strengthening agent can be internally added to a paper material or wet paper. Examples of the dry paper strengthening agent include starch, polyacrylamide, carboxymethyl cellulose (CMC) or sodium carboxymethyl cellulose as a salt of CMC, calcium carboxymethyl cellulose, and zinc carboxymethyl cellulose. Examples of the wet paper strengthening agent include a polyamide polyamine epichlorohydrin resin, a urea resin, an acid colloid/melamine resin, and a thermally crosslinkable coating PAM. Incidentally, when a dry paper strengthening agent is internally added, the amount thereof added to pulp slurry is about 1.0 kg/pulp t or less. The wet paper strength enhancer is desirably cationic, and the amount thereof added to pulp slurry is about 5.0 to 20.0 kg/pulp t.

The tissue paper according to the present embodiment can be manufactured by a similar method to a method for manufacturing conventional moisturizing tissue paper. That is, a single-layer tissue paper sheet having a crepe, made by a paper manufacturing machine, is wound to form a primary paper roll. The two primary paper rolls are set in a multi-ply forming machine also called a ply machine, and a single-layer continuous sheet is reeled out from each of the primary raw fabric rolls to be a multi-ply continuous sheet. Thereafter, the multi-ply continuous sheet is wound as a secondary paper roll, for example, by appropriately slitting the multi-ply continuous sheet. Then, using this secondary paper roll, a bundle is formed in a folding machine also called an interfolder or the like. For example, the bundle is cut into an appropriate size to obtain a product for the tissue paper. Then, by applying a moisturizing chemical liquid containing a moisturizing agent to tissue paper in any step in the manufacturing steps to obtain a product or in a chemicals applying step separately provided between the steps.

Next, particularly an effect of the tissue paper according to the present embodiment described above will be described in "Examples".

### Examples

A test sample of the tissue paper according to the present invention and a test sample of tissue paper different from the present invention were manufactured and subjected to the following sensory tests using "soft texture", "fullness", "wet feeling/moist feeling" as evaluation items for examination. A physical property value/composition value and the like of each sample were measured as follows. A physical property value/composition value and a test result of each sample are as illustrated in Table 1 below.

### [Basis weight]

Measurement was performed according to JIS P 8124 (1998). A value in Table indicates an average value of values for plies.

### [Paper thickness]

Measurement was performed according to the above thickness measuring method using a dial thickness gauge (thickness measuring instrument) "PEACOCK G type" (manufactured by Ozaki MFG. Co., Ltd.) under conditions of JIS P 8111 (1998).

### [Density]

A density is a value obtained by dividing a value (C) obtained by doubling the basis weight of tissue paper subjected to humidity control under conditions of JIS P 8111 (1998) by a paper thickness (D) of the above tissue paper (2-ply), represented in unit of g/cm³ to three decimal places.

### [Dry tensile strength]

Measurement was performed according to a tensile test of JIS P 8113 (1998).

### [Wet tensile strength]

Measurement was performed according to a tensile test of JIS P 8135 (1998).

### [Elongation percentage]

Measurement was performed according to a tensile test of JIS P 8113 (1998) using a "Multifunctional, Easy-to-Use Tensile and Compression Testing Machine TG-200N" manufactured by Minebea Inc.

### [Softness]

Measurement was performed by a handle-o-meter method according to a JIS L 1096 E method. However, a test piece had a size of 100 mm × 100 mm, and measurement was performed with a clearance of 5 mm. Measurement was performed five times in each of a longitudinal direction and a lateral direction with one ply, and an average value of all the ten values is represented in unit of cN/100 mm.

### [MMD]

While a contact surface of a friction contactor was brought into contact with a surface of a measurement sample to which a tension of 20 g/cm was applied in a predetermined direction at a contact pressure of 25 g, the measurement sample was moved by 2 cm in substantially the same direction as the direction in which the tension was applied at a speed of 0.1 cm/s. A friction coefficient at this time was measured using a friction sense tester KES-SE (manufactured by Kato Tech Co., Ltd.). This friction coefficient was divided by a friction distance (moving distance = 2 cm), and the obtained value was used as MMD. The friction contactor was formed by adjoining 20 piano wires P each having a diameter of 0.5 mm, and had a contact surface formed such that the length and the width were both 10 mm. On the contact surface, a unit bulging portion having the front end formed with 20 piano wires P (radius of curvature: 0.25 mm) was formed.

### [Content of chemicals]

The content of chemicals is the amount of the chemicals in a sample at the time of absolute dryness. Note that the term "time of absolute dryness" refers to the amount of chemicals in a state of being dried at a temperature of 65°C and a humidity of 10% until the chemicals obtain a constant mass.

### [Content ratio of chemicals (absolute dryness)]

The content ratio of chemicals (absolute dryness) is a ratio of the chemicals contained in a sample at the time of absolute dryness.

### [Content ratio of chemicals (containing moisture)]

The content ratio of chemicals (containing moisture) is a ratio of the chemicals (containing moisture) contained in a sample in a humidity-controlled state for 24 hours under conditions of a temperature of 23°C and a humidity of 50%. Note that the amount of chemicals is calculated from the mass of a sample in a humidity-controlled state, the mass of the sample at the time of absolute dryness, and the above contents of the chemicals.

### [Ratio of 1.3 propanediol in paper]

A ratio of 1.3 propanediol in paper is a ratio of the mass of 1.3 propanediol with respect to the mass of a sample at the time of absolute dryness.

### [Sensory test]

Using tissue paper containing no chemicals in Comparative Example 3 as a reference sample, in comparison with the reference sample, 30 evaluators evaluated samples as follows. That is, concerning "soft texture" , "fullness", and "wet feeling and moist feeling", a sample felt to be "very good" was evaluated as "5", a sample felt to be "good" was evaluated as "4", a sample felt to be "neither good nor poor" was evaluated as "3", a sample felt to be "poor" was evaluated as "2", and a sample felt to be "very poor" was evaluated as "1". An average score of scores by the evaluators was calculated to be used as an evaluation value. For "comprehensive evaluation", a sample felt to have "good texture to give very high intention for purchase" was evaluated as "5", a sample felt to have "good texture to give high intention for purchase" was evaluated as "4", a sample felt to have "normal texture to give neither high nor low intention for purchase" was evaluated as "3", a sample felt to have poor texture to give low intention for purchase" was evaluated as "2", and a sample felt to have very poor texture to give little intention for purchase" was evaluated as "1". An average score of scores by the evaluators was calculated to be used as an evaluation value. Note that Comparative Example 3 corresponds to base paper before a chemical liquid is applied in Examples 1 to 11 each of which contains chemicals are Examples of the present invention and Comparative Examples 4 to 10.

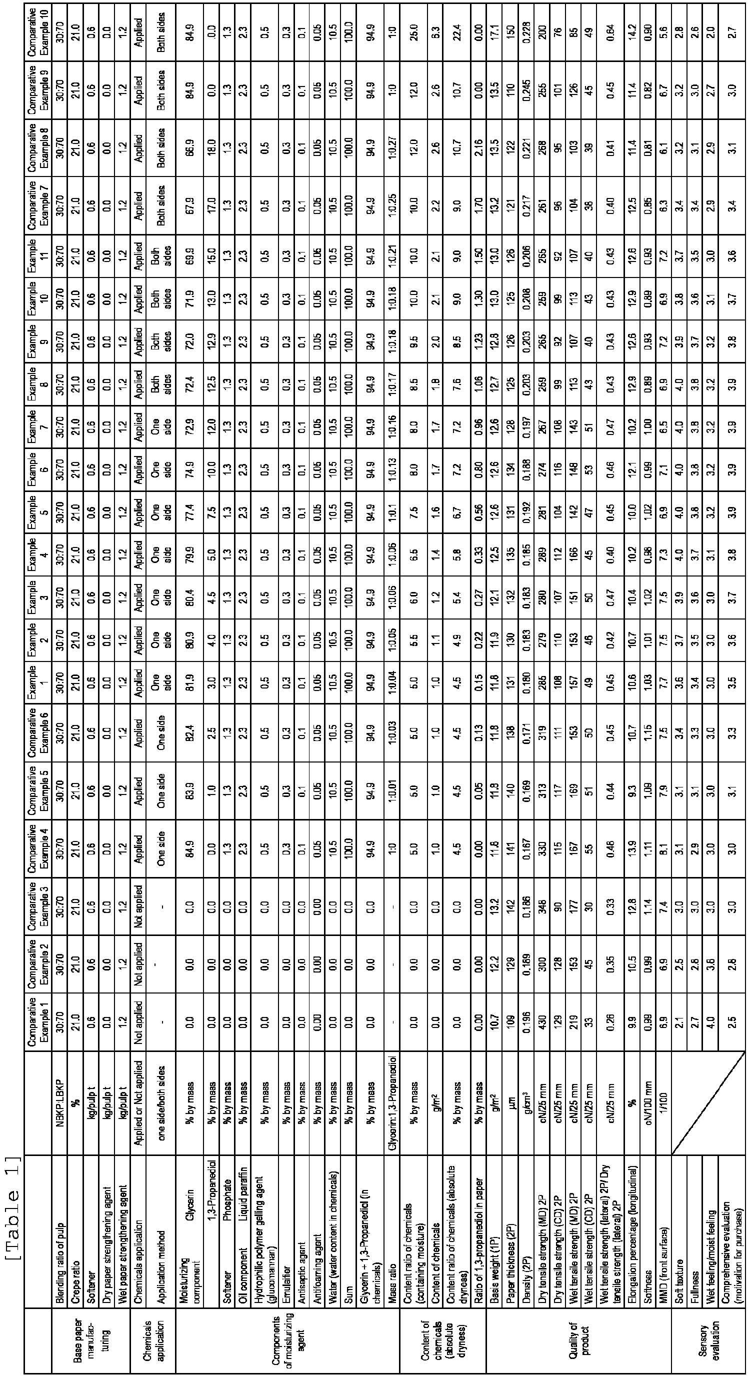

### [Test results]

Test results will be described with reference to Table 1 and Fig. 1 graphically illustrating sensory evaluation in Examples 1 to 7 and Comparative Examples 3 to 8 illustrated in Table 1. Note that the horizontal axis in Fig. 1 corresponds to a ratio of 1,3-propanediol in paper, and the vertical axis corresponds to sensory evaluation.

Among samples in Table 1, samples in Comparative Examples 1 to 3 are non-moisturizing tissue paper containing no moisturizing agent, and have different basis weights and paper thicknesses from one another. In comparison among these samples, as the "basis weight" and the "paper thickness" decrease, "wet feeling/moist feeling" is improved, but "soft texture" and "fullness" tend to decrease. However, any case gives low motivation for purchase.

Comparative Example 4 contains conventional chemicals containing only glycerin as a main effective component without containing 1,3-propanediol, that is, chemicals in which a ratio of 1,3-propanediol in paper is zero in an amount of 1.0 g/m². In Comparative Example 4, the content of the chemicals is set to a low value at approximately the same level as in Examples 1 and 2 while only glycerin is used as a main effective component as in related art. When Comparative Example 4 is compared with Comparative Example 3 as a reference sample, "wet feeling/moist feeling" does not decrease due to the low content of the chemicals. In addition, "soft texture" is slightly improved. However, in exchange therewith, it can be confirmed that "fullness" slightly decreases.

In Comparative Examples 5 and 6, the content of chemicals is the same as in Comparative Example 4, but chemicals containing glycerin and 1,3-propanediol are used. However, a ratio of glycerin and 1,3-propanediol in paper is 0.13% by mass or less, and a ratio of 1,3-propanediol in paper is lower than that in the present invention. Incidentally, in Comparative Examples 5 and 6, the ratio of 1,3-propanediol in paper is higher in Comparative Example 6. When Comparative Examples 4 to 6 are compared with one another, "fullness" is improved as the ratio of 1,3-propanediol in paper increases, and "soft texture" is also improved as the ratio increases. "Wet feeling/moist feeling" does not decrease, either. However, although "soft texture" and "fullness" are individually improved at such a ratio in paper as in Comparative Example 6, dramatic improvement cannot be confirmed, and comprehensive evaluation (motivation for purchase) is not increased so much.

Next, when these Comparative Examples 4 to 6 are compared with Example 1 of the present invention, in Example 1, the content ratio of chemicals is the same as that in Comparative Examples 4 to 6, but remarkable improvement in "soft texture" and "fullness" can be confirmed. It can be confirmed that comprehensive evaluation (motivation for purchase) is also increased.

Furthermore, refer to Examples 1 to 11. The ratio of 1,3-propanediol in paper is increased from Example 1 toward Example 11. Incidentally, the content of chemicals in Example 6 is the same as that in Example 7, and the content of chemicals in Example 10 is the same as that in Example 11. When these Examples are compared with one another, evaluation for "soft texture", "fullness", and "wet feeling/moist feeling" tends to be higher from Example 1 toward Example 2, Example 3, Example 4, and Example 5, and very high evaluation continues from Example 5 to Example 8. From Example 9, evaluation tends to fall slightly. Comprehensive evaluation (motivation for purchase) also peaks in Examples 5 to 8. In Examples 1 to 11, satisfactory results are obtained for MMD and softness.

Subsequently, when Example 11 is compared with Comparative Examples 7 and 8, in Comparative Example 7, the ratio of 1,3-propanediol in paper is higher than that in Example 11, and outside the range of the present invention. In Comparative Example 8, the ratio of 1,3-propanediol in paper is still higher than that in Comparative Example 7. When these results are compared with one another, in Comparative Example 7, the blending amount of glycerin is sufficient, and the ratio of 1,3-propanediol is also high, but evaluation of "soft texture" and "fullness" falls more significantly than that in Examples 10 and 11. Evaluation of "wet feeling/moist feeling" also falls sharply. Furthermore, in Comparative Example 11, the content of chemicals and the ratio of 1,3-propanediol are higher than those in Comparative Example 7. However, in Comparative Example 8, evaluation of "soft texture" and "fullness" falls sharply.

Furthermore, refer to Comparative Examples 9 and 10. Comparative Example 9 contains 2.6 g/m² of conventional chemicals containing only glycerin as a main effective component without containing 1,3-propanediol. Comparative Example 10 contains 6.3 g/m² of conventional chemicals containing only glycerin as a main effective component. In Comparative Example 9, "soft texture" is evaluated to be higher than Comparative Example 3 as a reference sample, "fullness" is evaluated to be equal to Comparative Example 3, and "wet feeling/moist feeling" is evaluated to be lower than Comparative Example 3. In Comparative example 10, all evaluation items are evaluated to be low. In Comparative Example 9, the content of chemicals is slightly lower than that of a commercially available product, but the content is higher than the level of Comparative Example 4. That is, it is indicated that conventional chemicals containing only glycerin as a main effective component cannot achieve a good balance among "soft texture", "fullness", and "sticky feeling". In Comparative Example 10, the content of chemicals is approximately the same as that of a conventionally commercially available product. However, the conventionally commercially available product usually has a higher basis weight than that according to the present invention. Therefore, it is considered that this has an influence.

From these facts, by adopting the configuration of the tissue paper of the present invention, remarkably excellent "soft texture" and "fullness" are achieved, a load on the skin is low even when the paper cleans the skin repeatedly, for example, even when the paper is frequently used for blowing one's nose, and furthermore, a feeling of applied chemicals such as "wet feeling", "moist feeling", or "sticky feeling" is remarkably reduced.

## Claims

1. Two-ply tissue paper,
wherein a basis weight per ply is 11.8 g/m² or more and less than 13.2 g/m² per ply,
a paper thickness in the two-ply state is more than 122 µm and less than 138 µm,
chemicals containing mainly glycerin and 1,3-propanediol are contained,
the chemicals having a mass ratio between the glycerin and the 1,3-propanediol of more than 1 :0.03 and less than 1 :0.25, are contained in an amount of 1.0 g/m² or more and less than 2.2 g/m² and
a ratio of 1,3-propanediol in the paper is more than 0.13% by mass and less than 1.70% by mass.

2. The two-ply tissue paper according to claim 1,
wherein MMD is less than 7.9 and softness is less than 1.09 cN/100 mm.

## Patentansprüche

1. Zweilagiges Tissue-Papier,
wobei ein Flächengewicht pro Lage 11,8 g/m² oder mehr und weniger als 13,2 g/m² pro Lage beträgt,
eine Papierdicke im zweilagigen Zustand mehr als 122 µm und weniger als 138 µm beträgt;
Chemikalien, die hauptsächlich Glycerin und 1,3-Propandiol enthalten, enthalten sind,
die Chemikalien mit einem Massenverhältnis zwischen dem Glycerin und dem 1,3-Propandiol von mehr als 1:0,03 und weniger als 1:0,25 in einer Menge von 1,0 g/m² oder mehr und weniger als 2,2 g/m² enthalten sind, und
ein Anteil von 1,3-Propandiol in dem Papier mehr als 0,13 Massen-% und weniger als 1,70 Massen-% beträgt.

2. Zweilagiges Tissue-Papier nach Anspruch 1, wobei die MMD weniger als 7,9 und die Weichheit weniger als 1,09 cN/100 mm beträgt.

## Revendications

1. Papier mince à deux plis,
dans lequel un grammage par pli est supérieur ou égal à 11,8 g/m² et inférieur à 13,2 g/m² par pli,
une épaisseur de papier à l'état deux plis est supérieure à 122 µm et inférieure à 138 µm,
des produits chimiques contenant principalement de la glycérine et du 1,3-propanediol sont contenus,
les produits chimiques ayant un rapport massique entre la glycérine et le 1,3-propanediol supérieur à 1/0,03 et inférieur à 1/0,25, sont contenus en une quantité supérieure ou égale à 1,0 g/m² et inférieure à 2,2 g/m² et
un rapport du 1,3-propanediol dans le papier est supérieur à 0,13 % en masse et inférieur à 1,70 % en masse.

2. Papier mince à deux plis selon la revendication 1, dans lequel un MMD est inférieur à 7,9 et une souplesse est inférieure à 1,09 cN/100 mm.
